# EUROPEAN PATENT SPECIFICATION

(11) **EP 4 583 953 B1**
(45) Date of publication and mention of the grant of the patent: **11.03.2026**
(21) Application number: 23783936.0
(22) Date of filing: 22.09.2023
(51) Int. Cl.: A61M 25/00, A61M 25/01

(54) **INTERMITTENT CATHETER**
INTERMITTIERENDER KATHETER
CATHÉTER INTERMITTENT

(30) Priority: 26.09.2022 US 202263409905 P; 26.09.2022 US 202263409923 P; 26.09.2022 US 202263409892 P; 28.10.2022 GB 202215966; 28.10.2022 GB 202215965; 28.10.2022 GB 202215967
(43) Date of publication of application: 16.07.2025
(73) Proprietor: ConvaTec Limited, Deeside, Flintshire CH5 2NU (GB)
(72) Inventor: KANDRÁC, Luká, 3 071 01 Michalovce (SK); LACKO, Patrik, 3 071 01 Michalovce (SK); NOVÁK, Marián, 3 071 01 Michalovce (SK)
(74) Representative: Wilson Gunn
(86) International application number: PCT/GB2023/052464
(87) International publication number: WO 2024/069137

(56) References cited:
- WO-A1-2019/245679
- WO-A2-2007/050685
- WO-A2-2009/054720
- US-A1- 2014 276 661
- US-A1- 2021 330 938
- US-A1- 2021 346 644

## Description

### Technical Field of the Invention

The present invention relates to an intermittent catheter (e.g. a urinary catheter).

### Background to the Invention

A catheter is a medical device comprising a hollow catheter tube designed for insertion into canals, vessels, passageways or body cavities to permit injection, drainage or withdrawal of fluids or substances therefrom, or to ensure said canals, vessels, passageways etc. remain open. Urinary catheters are designed for use for insertion into a user's bladder via the urethra to drain the bladder.

To maximise comfort and minimise the risk of trauma and/or infection, an outer surface of the catheter tube is typically wetted using a wetting agent prior to insertion by the user. In further developments, the catheter tube itself comprises, is integrated with or is coated with a hydrophilic component (e.g. a hydrophilic polymer) which serves to reduce friction further upon application of the wetting agent.

Some catheters may be supplied pre-wetted in a packaging, for instance, where the catheter is at least partially submerged within wetting agent within the packaging. Whilst this may ensure the catheter tube is adequately wetted prior to use, such arrangements suffer in that components of the catheter other than the catheter tube such as a gripper element or funnel can also become wetted. This has a detrimental effect of the experience of the user where it may become difficult to hold and direct the catheter tube as required. This is particularly problematic where the user is performing self-catheterisation. Further, having the catheter submerged may effectively reduce the shelf-life of the catheter due to long-term exposure of components of the catheter to moisture.

It is therefore seen advantageous to provide a catheter which may be wetted at or immediately prior to the point of use.

In an attempt to address this, some catheters are provided in packaging which includes a rupturable container or sachet within the packaging which a user may burst to release the wetting agent. Typicaly, this involves the user squeezing the packaging to cause the container/sachet to break. However, such arrangements experience similar problems to those discussed above where the wetting agent is allowed to come into contact with other components of the catheter. Such arrangements also result in the possibility of the catheter tube not being fully wetted, or indeed wetted at all, prior to use. This can be harmful for the user. Furthermore such systems may require a degree of dexterity and offer no feedback to ensure wetting has occurred.

It is therefore advantageous to provide a cathater which includes a means of supplying a wetting agent solely to the catheter tube to improve user experience.

In further prior art solutions, the catheter may be packaged within a packaging which includes a wetting device. In use, the catheter tube may be moved through the wetting device as the catheter is removed from the packaging and in doing so wetting the catheter tube. Examples of such catheters are shown in PCT application No. PCT/IB2018/001539 in the name of ConvaTec Limited.

Further examples can be found in US2021/0346644A1 and US2021/0330938A1. US2021/0346644A1 describes a wetting mechanism for wetting a tube of a catheter. The wetting mechanism includes a housing positioned initially at or proximal to the tip end of the catheter tube. The housing comprises a wetting chamber into which at least a portion of the catheter tube is able to be introduced and be moved therethrough to wet the catheter tube in use. The wetting mechanism includes a stopper which is moveable between a first and second positions to prevent or allow the tip end of the catheter tube to be moved through the wetting chamber. US2021/0330938A1 describes a wetting mechanism for wetting a tube of a catheter. The wetting mechanism includes a housing positioned initially at or proximal to the tip end of the catheter tube. The housing comprises a wetting chamber into which at least a portion of the catheter tube is able to be introduced and be moved therethrough to wet the catheter tube in use. The wetting mechanism includes a wetting applicator positioned within the wetting chamber configured to hold fluid therein and release said fluid to wet the catheter tube upon movement of the tube through the wetting chamber.

However, due to packaging constraints the amount of wetting agent able to be contained in such wetting devices is low, and there therefore remains a possibility of the catheter tube not being fully wetted in such solutions, especially where the catheter is near the end of its shelf life and some of the solution may have evaporated.

For mechanisms which wet the catheter tube from the distal end, an insufficient volume of wetting agent may result in the tip end not being wetted at all which is undesirable since the tip end will be introduced into the urethra first and is hence most likely to cause injury if inadequately wetted before use.

It is an aim of an embodiment or embodiments of the invention to overcome or at least partially mitigate one or more problems with the prior art and/or to provide an improved intermittent catheter.

### Summary of the Invention

The present invention provides a catheter assembly according to the appended claims.

An aspect of the present disclosure provides a catheter assembly comprising: an external housing comprising a main body and a cap. The external housing and cap may form a cavity for housing at least part of a catheter. The external housing may comprise a wetting agent storage chamber. The wetting agent storage chamber may be defined by a chamber wall and an inserter tip. The inserter tip may be movable with respect to the chamber wall between a first position and a second position. **In** the first position the inserter tip may be retracted. **In** the first position the inserter tip may be aligned with the chamber wall to seal the storage chamber. **In** the second position the inserter tip may be deployed. In the second position the inserter tip may be misaligned with the chamber wall to open the storage chamber. Removal of the cap may move the inserter tip from the first position to the second position.

An aspect of the present disclosure provides a catheter assembly comprising an external housing and cap; wherein the external housing and cap form a cavity for housing at least part of a catheter; the external housing comprising a wetting agent storage chamber defined by a chamber wall and an inserter tip, wherein the inserter tip comprises an insertion end configured to be inserted into a urethra and the cap comprises an interior cavity shaped to conform to a portion of the inserter tip, wherein the inserter tip is movable with respect to the chamber wall between a first position and a second position; wherein in the first position the inserter tip is retracted and is aligned with the chamber wall to seal the storage chamber; and in the second position the inserter tip is deployed and is misaligned with the chamber wall to open the storage chamber; wherein removal of the cap moves the inserter tip from the first position to the second position.

Advantageously, initiating opening of the wetting agent storage chamber (and thus wetting the catheter) and deployment of the inserter tip are both achieved by moving the cap. This means a user can be provided with a clear indication that wetting has occurred. Furthermore, it allows the device to be deployed ready to use in a single action. The cavity may be a sterile cavity.

The inserter tip may comprise one or more ribs extending circumferentially. The ribs may extend to the chamber wall. The inserter tip may comprise at least two ribs. The inserter tip may comprise at least one upper rib and at least one lower rib. For example, the inserter tip may comprise a pair of upper ribs and a pair of lower ribs. Alternatively (or additionally) the chamber wall may comprise one or more ribs extending circumferentially. The ribs may extend to the inserter tip. The chamber wall may comprise two ribs. One of the inserter tip or chamber wall preferably comprises one or more ribs extending circumferentially and projecting to the other of the chamber wall or insert.

In the first position the one or more ribs may define an axial extent of the wetting agent storage chamber. Where the inserter tip comprises upper and lower ribs, the distance between the upper and lower ribs may define the axial extent of the storage chamber. Where the chamber wall comprises two ribs, the two ribs may define the axial extent of the storage chamber.

In the first position one or more or each rib of the inserter tip preferably aligns with a corresponding sealing surface on the chamber wall to seal the storage chamber. Alternatively, in the first position one or more or each rib of the chamber wall preferably aligns with a corresponding sealing surface on the inserter tip to seal the storage chamber. In the second position at least one rib of the inserter tip is preferably axially misaligned with a sealing surface on the chamber wall. Where the assembly comprises upper and lower ribs, the upper rib may remain aligned with a sealing surface on the chamber wall in the second position. In an alternative, in the second position at least one rib of the chamber wall is axially misaligned with a sealing surface on the inserter tip.

The storage chamber may be sealed with a seal element, one or more of the ribs may comprise a seal element. The seal element may be integrally formed with the inserter tip. The seal element may be integrally formed with the external housing. The seal element may be mounted between a pair of ribs.

The seal element may be an elastomeric material. For example, the seal may be a rubber seal or some other suitable material. The seal element may be an annular seal element. The seal element may comprise an O-ring. The seal element may comprise an X-ring. The seal element may comprise a U-cup. The seal element may lie in a radial plane, for example, the normal plane of the longitudinal axis of the catheter assembly. The seal element may extend between radially opposed sealing surfaces. The O-ring may lie in a radial plane, for example, the normal plane of the longitudinal axis of the catheter assembly. The seal element may be provided in the form of a gasket.

The seal element may be compressible.

The seal element may be located in a seal element housing in one of the chamber wall or the inserter tip. The seal element housing may comprise a groove or channel provided within the chamber wall or inserter tip. The groove or channel may be provided in part by a radially outer wall, an axial end wall and/or one or more catheter guide features provided within the storage chamber. The seal element may be overmoulded so as to become an integral part of the storage chamber or inserter tip. One or more of the sealing surfaces may be provided by the seal element housing. The at least one sealing surface may slideably oppose the seal element. The at least one sealing surface may be referred to as a primary sealing surface.

At least one of the inserter tip and the chamber wall may comprise a divergent portion over which the rib passes when transitioning between the first position and second position such that the distance between the chamber wall and inserter tip at the axial location of the rib is increased when in the second position when compared to the first position. The distance may be a radial distance with respect to the longitudinal axis. The divergent portion may comprise a widening of a cavity adjacent to the seal surface. The cavity may be that of the storage chamber and/or an adjacent chamber. The adjacent chamber may be provided within the external housing. The adjacent chamber may house a catheter tube. The adjacent chamber may be a priming chamber or a wetting chamber.

The divergent portion may comprise a step, taper or chamfer in the wall surface which is adjacent to the sealing surface. The sealing surface may comprise a cylindrical surface having a first diameter. An adjacent wall portion of the chamber wall or inserter tip may have a second diameter which is different to the first diameter. The first diameter may be greater than the second diameter. The adjacent wall portion may be proximal an insertion end of the catheter. The adjacent wall portion may be a secondary surface.

The catheter assembly may further comprise a wetting agent. In the first position the wetting agent may be sealed within the wetting agent storage chamber. When in the second position, a flow path may be provided between the sealing surface and rib such that the wetting agent may flow out of the storage chamber when in the second position. The flow path may direct the wetting agent out of the storage chamber to the catheter.

The catheter assembly may further comprise a wetting chamber. In the second position the wetting chamber may be in fluid communication with the storage chamber. The storage chamber and wetting chamber may be separate chambers.

The catheter assembly may further comprise a sheath. The wetting chamber may be at least partially defined by the sheath. The sheath may define a cavity. The catheter may be arranged in the sheath cavity. In the second position the sheath cavity may be in fluid communication with the wetting agent storage chamber. In this regard the sheath cavity may be a wetting chamber.

The catheter may be at least partially arranged within the sheath. The sheath and catheter may define a flow path. The flow path may extend along the axial length of the catheter. The flow path may be provided between an outer surface of the catheter and an inner surface of the sheath.

The catheter assembly may further comprise a storage pouch. The storage pouch may be configured to receive fluid discharge from the catheter in use. In the second position the storage pouch may be in fluid communication with the wetting agent storage chamber. The storage pouch may be in fluid communication with the wetting agent storage chamber via the sheath flow path.

When in the second position, the seal may be separated from at least one of the sealing surfaces so as to provide a flow path for the wetting agent to pass the rib.

The wetting agent may be provided within, or capable of flowing into, the wetting chamber when in the second position.

The inserter tip may comprise an inserter stop. The inserter stop may limit the extent to which the inserter tip can be inserted into a urethra. The inserter tip may be irreversibly moved to the deployed position. The external housing may comprise a deformable aperture though which the inserter tip is extends from an interior of the catheter body assembly to an exterior of the catheter body assembly. The inserter tip may comprise one or more projections of greater dimensions than the aperture. On deployment of the inserter tip the aperture may be deformed to allow the projections to move from an interior of the external housing to an exterior. After deployment the aperture may restrict movement of the one or more projections into the interior of the external housing.

Advantageously, the provision of projections on the inserter tip can be used to both limit the insertable length of the inserter tip to ensure user comfort and also to ensure that the movable inserter tip does not retract into the external housing. In cases where the inserter tip could retract there may be a risk of insufficient inserter tip being available hindering insertion of the catheter.

Accordingly, in a preferred embodiment of the present disclosure there is provided a catheter assembly comprising an external housing and a cap; the external housing comprising a wetting agent storage chamber defined by a chamber wall and an inserter tip, wherein the inserter tip is movable with respect to the chamber wall between a first position and a second position; wherein in the first position the inserter tip is retracted; and in the second position the inserter tip is deployed; wherein the external housing comprises a deformable aperture through which the inserter tip extends from an interior of the external housing to an exterior of the external housing, and the inserter tip comprises one or more projections of greater dimensions than the aperture wherein after deployment the aperture may restrict movement of the one or more projections into the interior of the external housing

The catheter assembly may comprise a plurality of seals. The plurality of seals may comprise a proximal seal which is proximal to an insertion end of the catheter. The plurality of seals may comprise a second seal. The second seal may be axially separated from the proximal seal on the distal side thereof. The distal seal and proximal seal may be located radially between the movable inserter tip and chamber wall. The distal seal and proximal seal may comprise respective distal and proximal seal elements and sealing surfaces.

The first seal may be arranged on or proximal to the inserter tip. The second seal may be arranged on the inserter tip.

The distal seal may maintain sealing contact in the first and second positions.

The catheter may comprise a catheter tube which provides an insertion end for inserting into a patient and an outlet end comprising one or more external handling features.

The catheter may be an intermittent catheter. The catheter may be a urinary catheter. The catheter may be a female intermittent catheter.

The catheter assembly may be configured to provide mechanical feedback to a user. The mechanical feedback may be indicative of the end of a first step. The mechanical feedback may be indicative of the catheter assembly being in the second position. The mechanical feedback may be between the first and second steps.

The provision of a feedback mechanism allows the user to know that the wetting process has occurred without having to visually inspect the catheter.

The catheter may comprise an insertable portion. Prior to the first step the insertable portion may not be in contact with the wetting agent.

It is advantageous that prior to deployment of the catheter, for example during shipping and storage, that the insertable portion of the catheter is not in contact with the wetting agent. The insertable portion of the catheter is often made of materials which interact with the wetting agent, this interaction should only occur just prior to use.

The inserter tip may further comprise an insertion guide.

The inserter tip may be provided on an inserter. The inserter may comprise a catheter gripper. The term catheter gripper should be understood as a part of the inserter which grips the catheter (not a part used by a user to grip the catheter). The inserter may be tubular. The catheter gripper may be arranged at one end of the inserter. The catheter gripper and inserter tip may be provided on opposing ends of the inserter.

The catheter gripper may engage with the catheter when the inserter is in the first position. The catheter gripper may disengage with the catheter when the inserter is in the second position.

Accordingly, in a further aspect of the present disclosure there is provided a catheter assembly comprising; a catheter assembly body, a movable insert and a catheter tube. The catheter assembly body may comprise a seat. The movable insert may comprise a tubular body. The tubular body may have a deformable first end. **In a** first position the movable insert may be arranged radially within the catheter assembly body. In a first position the catheter tube may be arranged radially within the movable insert. In a first position the seat may bear against the first end of the movable insert. In the first position the first end of the movable inset may be deformed such that the first end of the movable insert grips the catheter tube. In a second position the movable insert may be disengaged from the seat such that the first end of the movable insert releases the catheter tube.

In a further aspect of the present disclosure there is provided a catheter assembly comprising: a catheter assembly body, the catheter assembly body comprising a seat; a movable insert, the movable insert comprising a tubular body with a deformable first end; and a catheter tube; wherein in a first position the movable insert is arranged radially within the catheter assembly body and the catheter tube is arranged radially within the movable insert, and wherein the seat bears against the first end of the movable insert, deforming the first end of the movable insert such that the first end of the movable insert grips the catheter tube; and wherein in a second position the movable insert is disengaged from the seat such that the first end of the movable insert releases the catheter tube.

The catheter assembly body may be the external housing. The movable insert may be an inserter or inserter tip.

The deformable first end may be resiliently deformable.

Accordingly, in a further aspect of the present disclosure there is provided a catheter assembly comprising: a catheter assembly body, the catheter assembly body comprising a seat; a movable insert, the movable insert comprising a tubular body with a resiliently deformable first end; and a catheter tube; wherein in a first position the movable insert is arranged radially within the catheter assembly body and the catheter tube is arranged radially within the movable insert, and wherein the seat bears against the first end of the movable insert, deforming the first end of the movable insert such that the first end of the movable insert grips the catheter tube; and wherein in a second position the movable insert is disengaged from the seat such that the first end of the movable insert releases the catheter tube.

Advantageously, by gripping the catheter whilst the assembly is in a first position, the position of the catheter relative to the wetting agent storage chamber can be ensured and this can improve consistency in the wetting process. Furthermore, it hinders or restricts the deployment of the catheter until the wetting agent storage chamber has been opened (and thus the catheter is wetted).

The deformable first end may be formed by structurally weakening the tubular body. The first end may have thinner walls than the remainder of the tubular body. The deformable first end may comprise one or more slots. The one or more slots may provide a gap into which the first end may deform. The first deformable end may comprise two slots. The two slots may define gaps between two arms. In the first position the two arms may bear against the seat the reduce the separation between the two arms. The arms may comprise a projection to bear against the seat. The two arms may be provided with apertures. The apertures may extend between a radially inner face which engages with the catheter and a radially outer face which engages with the seat.

The seat may comprise a divergent portion over which the deformable first end passes when transitioning between the first position and second position such that the distance between the movable insert and seat at the axial location of the rib is increased when in the second position when compared to the first position. The distance may be a radial distance with respect to the longitudinal axis. The divergent portion may comprise a widening of a cavity adjacent to the seat. The cavity may be that of the storage chamber and/or an adjacent chamber. The adjacent chamber may be provided within the catheter assembly body. The adjacent chamber may house the catheter tube. The adjacent chamber may be a priming chamber or a wetting chamber.

The divergent portion may comprise a step or chamfer.

The catheter assembly may further comprise a wetting agent storage chamber. The wetting agent storage chamber may be defined by the catheter assembly body and the movable insert. In the first position the wetting agent storage chamber may be sealed. In the second position the wetting agent storage chamber may be open.

The catheter tube may be gripped with an insertion end proximal to an opening in the wetting agent storage chamber.

The movable insert may be movable along an axis relative to the catheter assembly body between the first position and the second position.

The/a cap may be configured to disengage from the inserter tip in the second position.

Accordingly, in a further aspect of the present disclosure there is provided a catheter assembly comprising a cap, a catheter assembly body and a movable insert. The cap may comprise a tubular body. The cap may have a closed end. The cap may have an open end. The open end may be deformable. The catheter assembly body may comprise a seat. The cap and movable insert may be configured to move along an axis relative to the catheter body between a first position and a second position. In the first position the open end of the cap may be restrained against the seat. In the first position the open end of the cap may be restrained against the movable insert. In the first position the cap and movable insert may be coupled together. In the second position the open end of the cap may be displaced from the seat. In the second position the cap and movable insert may decouple.

Accordingly in a further aspect of the present disclosure there is provided a catheter assembly comprising: a cap, the cap comprising a tubular body with a closed end and an open end, wherein the open end is deformable; a catheter assembly body comprising a seat; and a movable insert; wherein the cap and movable insert are configured to move along an axis relative to the catheter body between a first position and a second position; wherein in the first position the open end of the cap is restrained against the seat and the movable insert such that the cap and movable insert are coupled together; and in the second position the open end of the cap is displaced from the seat such that the cap and movable insert decouple.

Advantageously, by making the cap and movable insert coupled, the wetting operation can be initiated in the same action as removing the cap. Furthermore, as the cap and moveable insert only decouple in the second position, the cap can only be removed once in the second position. Movement to the second position may cause wetting of the catheter and as such the cap may only be removed once the wetting action has occurred, therefore reducing the risk of injury to the user associated with use of an unlubricated catheter.

The cap may comprise a deformable portion. The cap may be formed of a resiliently deformable material. The cap may be formed of linear low-density polyethylene. The cap may be formed of low-density polyethylene. The cap may be formed of polypropylene. The cap may be formed of a thermoplastic elastomer. The cap may be formed of thermoplastic polyurethane. The cap may be formed of Ethylene-vinyl acetate.

The cap may be resiliently deformed in the first position. The cap may relax to an original shape in the second position. The cap may alternatively be deformed in the second position.

The cap may comprise a grab loop. The grab loop may be shaped to aid in removal of the cap.

The open end of the cap may be deformable. The open end of the cap may be formed of a deformable material. The open end of the cap may be provided with one or more features to aid in deformation. For example, the open end of the cap may comprise one or more slots extending axially from the open end. The deformation aids may be two slots. The open end of the cap may comprise one or more engagement tabs. The cap may be provided with two engagement tabs. The one or more engagement tabs may extend axially from the open end. The engagement tabs may be configured to flex radially.

The cap and movable insert may comprise corresponding engagement means. The movable insert may be an inserter tip. The engagement tabs may engage with the inserter tip. The inserter tip may comprise projections. The moveable insert may comprise recesses. The corresponding engagement means, e.g. projections and recesses, may be configured to remain engaged when subjected to the first predetermined force and configured to disengage when subject to the second predetermined force.

The engagement means may comprise projections on the movable insert and corresponding apertures on the cap. When the cap and movable insert are engaged the projections may be arranged in the apertures. The cap may be deformed to remove the projections from the apertures. The cap may be removed to decouple from the movable insert.

The catheter assembly may comprise a hermetic seal for preserving the sterility of the internal volume prior to use. In the first position the cap may provide a sterile seal for a cavity within the catheter assembly body.

When in a sealed configuration, the hermetic seal may be intact. When in a primed or wetting configuration, the hermetic seal may be broken such that the cavity within the catheter assembly body is at least partially open to the external air such that sterile environment may be considered breached.

The catheter assembly may further comprise an aperture through which the movable insert extends from an interior of the catheter body assembly to an exterior of the catheter body assembly. The aperture may be the seat.

The catheter tube may be functionalised. For example it may comprise, be integrated with or be coated with a hydrophilic component (e.g. a hydrophilic polymer). The hydrophilic component serves to reduce friction further upon application of the wetting agent. At least an external surface of the catheter tube may be functionalised, e.g. the hydrophilic component may be provided on at least an external surface of the catheter tube (which is in contact with the urethra in use). The catheter may comprise a main flow path for the passage of urine. The main flow path may extend along and define a longitudinal axis of the catheter. The main flow path may be provided by a wall of catheter tube. The main flow path may have a proximal inlet at an insertion end of the catheter, and a distal outlet.

The catheter may comprise an outlet body. The outlet body may incorporate the terminal end of the catheter tube. The outlet body may comprise the external handling surface of the catheter. The outlet body may comprise one or more flow enhancing features for aiding the flow from catheter tube. The one or more flow enhancing features may comprise a funnel, for example.

The outlet body may comprise or be referred to as a connector which connects the outlet end, e.g. a funnel and/or the external handling features, and the catheter tube.

The sheath may be a retractable sheath. The retractable sheath may be configured to be retracted during insertion of the catheter such that it provides a temporary enclosure around the catheter tube prior to insertion.

According to a further aspect of the of the present disclosure there is provided a catheter assembly comprising an external housing and an inserter tip. The external housing may comprise a deformable aperture. The inserter tip may extend through the aperture from an interior of the external housing to an exterior of the external housing. The inserter tip may be movable. The inserter tip may be movable between a retracted position and a deployed position. The insert tip may comprise one or more projections. The one or more projections may be of greater dimensions than the aperture. After deployment the aperture may restrict movement of the one or more projections into the interior of the external housing.

According to a further aspect of the present disclosure there is provided a catheter assembly comprising an external housing and an inserter tip, wherein the external housing comprises a deformable aperture through which the inserter tip extends from an interior of the external housing to an exterior of the external housing, wherein the inserter tip is movable between a retracted position and a deployed position; and the inserter tip comprises one or more projections of greater dimensions than the aperture wherein after deployment the aperture may restrict movement of the one or more projections into the interior of the external housing.

The inserter tip may comprise at least two projections. The insert tip may comprise at least three projections. The insert tip may comprise at least four projections. The projections may project radially outward from the insert tip.

The one or more projections may have a rectangular cross section. A long edge of the rectangular cross section may be aligned perpendicular to a longitudinal axis of the catheter assembly.

The one or more projections may comprise a radially outer face. The one or more projections may have a radial extent. The radial extent may be defined as the distance between the radially outer face and a radial centre of the inserter tip. The radial extent of the projections may be greater than the radius of the aperture. Where the catheter assembly comprises two projections, these may be arranged on opposing sides of the inserter tip. The separation between the radially outer faces of the two projections may be greater than the diameter of the aperture.

The catheter assembly may further comprise one or more chamfered sections. The chamfered sections may be configured to deform the aperture. The chamfered sections may be configured to deform the aperture as the inserter tip is moved from the retracted position to the deployed position. The aperture may be resiliently deformed.

The one or more projections may comprise the chamfered sections. The one, or more, each projections may comprise a leading face. The leading face may be the face of the projection facing which in use faces the urethra. The one, or more, or each projection may comprise a chamfer between the leading face and radially outer face.

The projections may be arranged between 1 and 20 mm from the insertion end. The projections may be arranged between 5 and 15 mm from the insertion end. The projections may be arranged between 8 and 12 mm from the insertion end.

The catheter assembly may further comprise a cap. The cap may engage with the inserter tip. The cap may engage with the one or more projections. The cap may engage with the one or more projections when the inserter tip is in the retracted position. The cap may be disengaged with the one or more projections when the inserter tip is in the deployed position. The cap may comprise one or more slots through which the projections extend. The cap may comprise a number of slots equal to the number of projections. The cap may comprise one or more recesses into which the projections extend. The cap may comprise a number of recesses equal to the number of projections.

The cap may be configured to move axially. The inserter tip may be configured to move axially. The axial movement of the cap may be transferred to the inserter tip. The axial movement of the cap may be transferred to the inserter tip via the slots and projections.

The cap may comprise the chamfered section. The chamfered section may be arranged between a narrow section and a wide section. The narrow section may have a radius less than the radius of the aperture. The wide section may have a radius approximately equal to or greater than the radial extent of the projections. The chamfered section may be arranged in the interior of the external housing when the inserter tip is in the retracted position. The chamfered section may be arranged to the exterior of the external housing when the inserter tip is in the deployed position.

Optional features set out above may apply to any aspect of the invention.

### Detailed Description of the Invention

In order that the invention may be more clearly understood one or more embodiments thereof will now be described, by way of example only, with reference to the accompanying drawings, of which:
Figure 1 shows a perspective view of a catheter assembly according to an embodiment of the disclosure;
Figure 2 shows a longitudinal sectional view of the catheter assembly of Figure 1 in a closed or sealed configuration;
Figure 3 shows an enlarged longitudinal section of the catheter assembly of Figure 1 detailing a storage chamber;
Figure 4 shows an enlarged longitudinal sectional view of the catheter assembly of Figure 1 detailing a cap; and
Figures 5a to 5c show a sequence of longitudinal sections of the catheter assembly of Figure 1 highlighting the wetting procedure for the catheter.

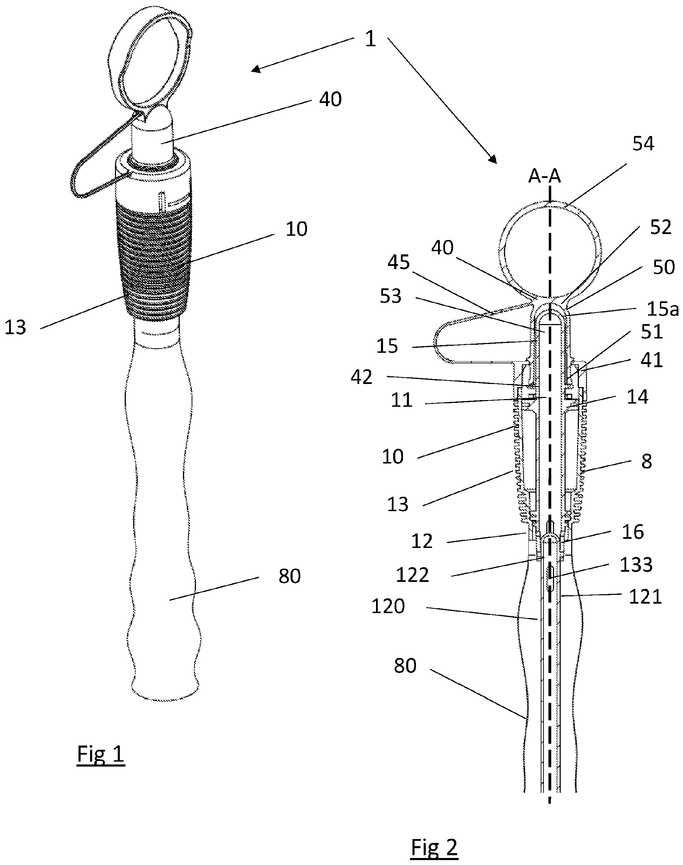

With reference to figures 1-5, an examplary catheter assembly 1 according to the present invention is described.

Referring to figures 1 and 2, the catheter assembly 1 comprises an external housing 10, a cap 40, a sheath 80 and a catheter 120. The catheter assembly 1 may be configured such that the catheter 120 may be wetted prior to being withdrawn from the external housing 10. A wetting mechanism may be provided within the external housing 10.

The external housing or catheter assembly body 10 has a tubular shape with a radially outer surface 8, a radially inner surface 9, an open cap end 11 and an open catheter end 12. The outer surface 8 of the external housing comprises a textured portion 13 to improve grip, in this embodiment provided by a plurality of ribs extending circumferentially around and radially outward of the outer surface 8. The textured portion 13 extends from the cap end 11 to the catheter end 12. In some embodiments the textured portion 13 may be integrally formed with the outer surface 8, in other embodiments the textured portion 13 may be formed of a different material, for example one which provides increased grip when wet.

Within the external housing 10 and projecting from each open end 12,13 is an inserter 14, the inserter having an overall tubular shape and comprising an inserter tip 15 and a catheter gripper 16. The external housing 10 and inserter 14 are concentrically arranged such that the inserter 14 is located within the external housing 10 in a radially nested configuration. The inserter tip 15 is provided at a first end of the inserter 14 and closes the tubular body with a hemispherical end 15a, the hemispherical end 15a is provided with one or more apertures or openings such as slits (not shown) which allow the end 15a to be opened to allow passage of a catheter therethrough. The opposite end of the inserter 14 is provided with the catheter gripper 16, the structure and operation of which will be elaborated upon below.

In this embodiment the inserter 14 is arranged within the external housing 10 such that in the storage position (as will be elaborated upon below) the inserter tip 15 extends out of the cap end 11 and the catheter gripper 16 extends out of the catheter end 12 of the external housing 10.

In this embodiment the cap 40 is formed in two parts, a connecting part 41 and a removable part 50. The connecting part 41 has an annular shape with an aperture 42 through the centre, it is connected to the cap end 11 of the external housing 10. The removable part 50 has a tubular shape with an open end 51 and a closed end 52, the interior of the removable part 50 defines a cavity 53 shaped to receive the inserter tip 15. The cavity may be sized and shaped to closely conform to the inserter tip 15. The exterior of the removable part 50 is shaped to engage with the aperture 42 in the connecting part 41. On the exterior of the removable part 50 at the closed end 52 there is provided a grab loop 54 by which a user can grip the cap 40. The two parts 41,50 of the cap 40 are connected together by a retaining strap 45.

The catheter 120 is of a type known to the art, comprising a catheter tube 121 with an insertion end 122 and an opposing drainage end (not shown). The catheter tube 121 has a longitudinal axis A-A which can be taken to be the principal axis of the catheter assembly 10. Proximate to the insertion end 122 there may be provided one or more drainage apertures 133, in this embodiment the drainage apertures 133 are oval in shape with the major axis being parallel with the principal axis A-A. It will be appreciated that the size and shape of the drainage apertures 133 may differ.

The exterior surface of the catheter tube 121 may be, as is known in the art, functionalised such that when wetted by the wetting agent the co-efficient of friction of the catheter tube 121 is reduced. The outer surface of the catheter tube 121 may be comprised of, or coated in, a functionalising material; for example the outer surface of the catheter tube 121 may have hydrophilic properties. The hydrophilic properties serve to reduce the coefficient of friction of the outer surface when the wetting agent is introduced.

The catheter 120 and inserter 14 are concentrically arranged such that the insertion end 122 of the catheter 120 engages with the catheter gripper 16 with the distal end of the catheter 120 extending away from the inserter 14 as will be described in further detail below.

Arranged at the catheter end 12 of the external housing 10 is the sheath 80. The sheath 80 extends away from the external housing 10 so as to surround at least a part of the catheter tube 120. The sheath 80 is formed of a flexible material. In some embodiments, the sheath 80 is attached to the external housing in such a way as to provide a leak resistant seal. In some embodiments the distal end of the sheath (not shown) may be joined to a pouch for collection of the fluid discharged from the catheter, alternatively the sheath 80 and pouch may be integrally formed. The sheath may allow for fluid communication from the external housing 10 to the pouch.

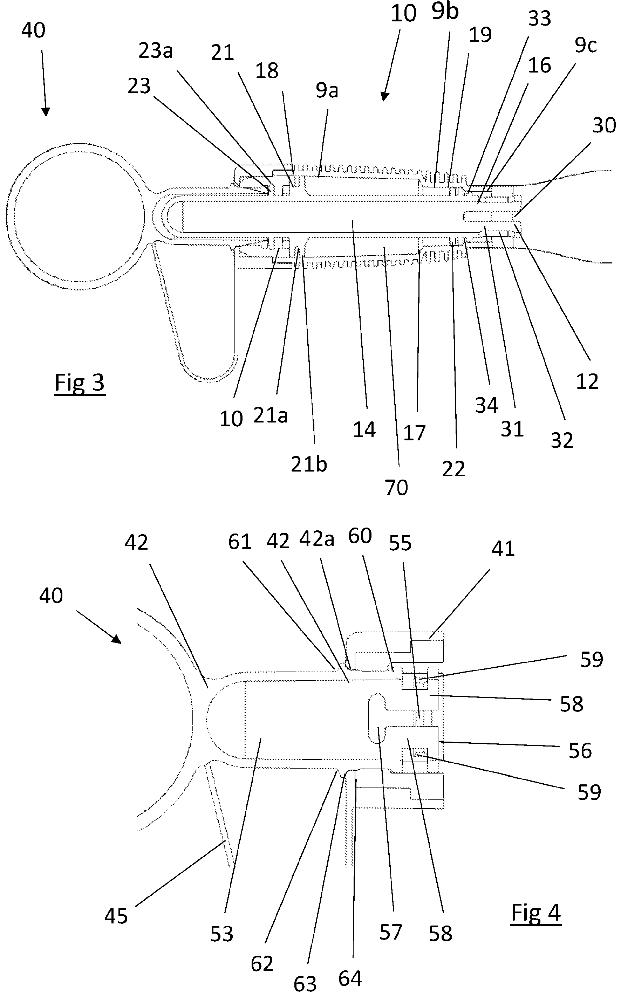

Referring to Figures 3 and 4, the arrangement of the external housing 10, inserter 14 and cap 40 are described in greater detail. The radially inner wall 9 of the external housing 10 is provided with a step proximate to the catheter end 12 such that the diameter of the inner wall 9 is reduced proximate to the catheter end 12. The first, wider, portion 9a of the inner wall 9 provides a first sealing surface 18 and the second, narrower, portion 9b of the inner wall 9 provides a second sealing surface 19. The internal diameter of the inner wall 9 is further reduced at the catheter end 12 edge by a further step 34 to form a third, narrowest, portion 9c.

The inserter 14 is radially nestled with the external housing 10. In this embodiment to ensure the alignment of the inserter 14 in the external housing the inserter 14 is provided with two pairs of ribs 21,22. In other embodiments the external housing may be provided with projections such as ribs.

In this embodiment a first (upper) pair of ribs 21 project circumferentially around, and radially outward, from the inserter 14 at a point approximately one third of the distance from the inserter tip 15a to the catheter gripper 16. The first pair of ribs 21 are sized such that they extend radially to the first sealing surface 18 of the external housing 10, the first pair of ribs 21 are formed from a single broader projection, the outer circumference of which is provided with a groove 21a with a depth equal to have the radial extent of the projection, this separates the projection into two equally sized ribs 21.

In this embodiment a second (lower) pair of ribs 22 project circumferentially around, and radially outward, from the inserter 14 at a point proximate to the catheter gripper 16. The second pair of ribs are sized such that they extend radially to the second sealing surface 19 of the external housing 10. The two ribs 22 of the second pair project separately from the inserter 14.

In this embodiment proximate to the first pair of ribs 21 are provided two cap tabs 23, the cap tabs 23 are two projections on opposing sides of the inserter 14. The cap tabs 23 have a rectangular base with the major edge normal to the principal axis A-A of the catheter assembly 10. Defining a radial extent of the cap tabs 23 is a radially outer face 23a.

In this embodiment at the catheter gripper 16 end of the inserter two slots 30 extend from the edge of the inserter 14 parallel to the principal axis A-A of the catheter assembly 10. The two slots 30 define a gap between two gripper arms 31, the gripper arms 31 are configured to engage with the catheter tube 121 when the assembly 10 is in a storage configuration as will be expanded upon below. Each of the gripper arms 31 are provided with an aperture 32 which extends from the radially inner surface to the radially outer surface.

By providing slots 30 in the inserter 14 the structural rigidity of the inserter 14 proximate to the gripper arms 31 is reduced and the gripper arms 31 are deformable to vary the separation between the two arms 31. Arranged at the base of each arm 31 on the radially outer side is a small bulge 33.

As noted above, the cap 40 is formed in two parts with the inner surface of the connector part 41 attached to the outer surface of the cap end 11 of the external housing 10. The connector part forms the end wall 64 of the external housing through which the aperture 42 is provided. In this embodiment the connector part 41 and external housing 10 are joined with a cantilever snap-fit connection, with corresponding projections and recesses on the connector part 41 and external housing 10. In. other embodiments other methods of attachment may be used for example, adhesive, welding or interference fit.

In this embodiment the open end 51 of the removable part 50 is provided with a pair of slots 55 on opposing sides of the opening. The slots 55 extend from the open lip 56 of the open end 51 approximately one third of the way to the closed end 52. The slots 55 widen at their closed end 57 such that they have a "T" shape. The two slots 55 define a gap between two engagement tabs 58.

The two engagement tabs 58 are each provided with a rectangular aperture 59 the long axis of which extends circumferentially around the cap. The apertures 59 are sized and shaped to receive the cap tabs 23 of the inserter 14. About the apertures 59 the thickness of the wall of the connector part 41 increases with a chamfered step 60 provided on the radially outer surface. The chamfered step 60 is sized such that the diameter at the widest point (that adjacent to the apertures 59) is approximately equal to the distance between the two radially outer faces 23a of the cap tabs 23.

In this embodiment a housing seal 61 is provided on the radially outer surface of the removable part 42 at a point corresponding approximately to half the axial extent of the cavity 53. The housing seal 61 comprises a sloped projection 62 extending circumferentially around the removable part 42, the side proximal to the open end 51 is provided with a small indent 63.

When provided in the sealed configuration, for example for storage and shipping the inserter 14 is provided in a retracted position within the external housing 10. The first and second pairs of ribs 21, 22 bear against the sealing surfaces 18,19 of the radially inner wall 9 to align the inserter 14 within the external housing 10 such that they are radially nested.

Together the inner wall 9 of the external housing 10, outer surface of the inserter 14, and ribs 21,22 define a wetting agent storage chamber 70, the inserter 14 and external housing 10 defining the radial extent and the upper and lower ribs 21,22 the axial extent. The wetting agent storage chamber 70 contains a wetting agent, which in this embodiment is water.

In the sealed configuration the inserter 14 is inserted to the maximum extent into the external housing 14, and the second pair of ribs 22 abut the further step 34 restricting any further insertion.

The removable part 50 of the cap 40 is arranged on the inserter 14 such that the inserter tip 15 is within the cavity 53. The cap tabs 23 project into the apertures 59 in the engagement tabs 58 and the open lip 56 abuts the first pair of ribs 21.

The removable part 50 of the cap 40 is also arranged through the aperture 42 in the connecting part 41; the housing seal 61 abuts the aperture 42 with a lip 42a of the aperture engaging with the indent 63. The housing seal 61 and aperture 42 provide a hermetic seal to ensure the sterility of the interior of the external housing 10.

During storage and transport the catheter 120 is retained within the external housing 10 by the catheter gripper 16. The insertion end 122 is nested between the gripper arms 31. The small bulge 33 has a greater diameter than the surrounding third inner wall portion 9c, the gripper arms 31 are therefore deformed radially inwards and bear against the insertion end 122 holding the catheter 120 in place. Securing the catheter 120 during storage is advantageous as it ensures the catheter 120 is in an optimal position prior to wetting and also that the catheter cannot be deployed without having been wetted.

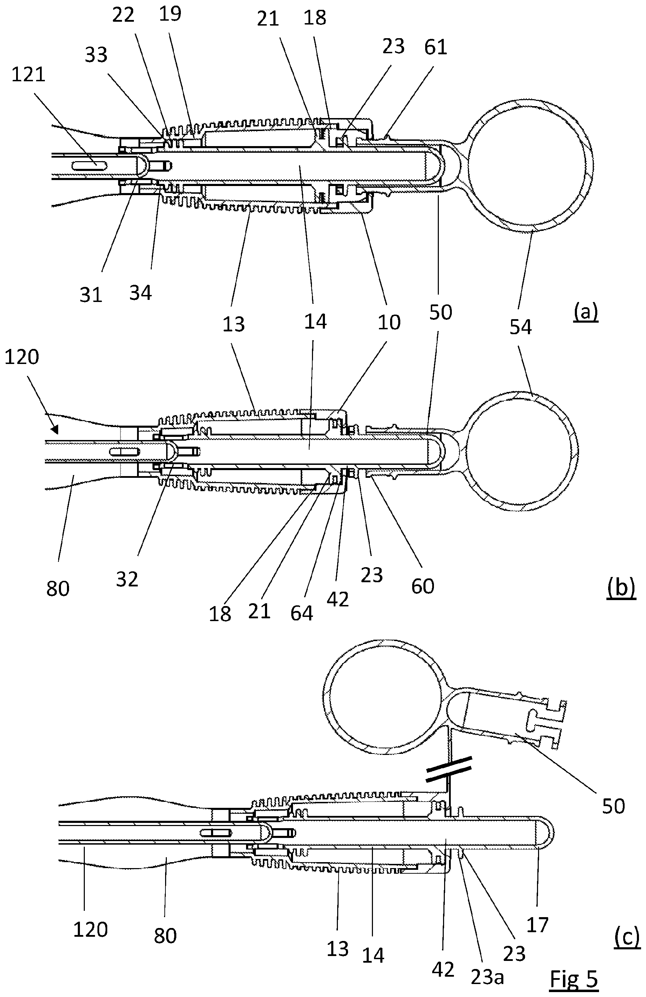

With reference to figures 5(a)-(c) the deployment of the catheter for use is described. Although shown on its side in figures 5(a)-(c), during deployment, the catheter assembly is arranged generally vertically, with the cap 40 uppermost (as shown in figures 1 and 2), so that the catheter 120 is below the wetting agent storage chamber 70. Gripping the textured portion 13 and the grab loop 54 a user pulls the removable part 50 of the cap 40 axially away from the external housing 10. As the removable part 50 is displaced axially away from the external housing 10, the aperture lip 42a disengages from the indent 63 thereby breaking the housing seal 61; the catheter assembly 10 is no longer hermetically sealed.

With continued axial displacement, the cap tabs 23 abut the edge of their corresponding apertures 59, with this the inserter 14 begins to be axially displaced. As shown in figure 5(a), as the inserter 14 is displaced the small bulge 33 passes the step 34 and becomes axially misaligned with the third inner wall portion 9c. As the small bulge 33 is no longer pressed against the third inner wall portion 9c the gripper arms 31 are no longer retained in a deformed position and relax in a radially outward manner increasing the separation between the two arms 31. This increase in separation results in the gripper arms 31 disengaging with the catheter 120 such that the catheter is free to be moved and the entire functionalised surface of the catheter tube 121 is exposed. Movement of the inserter 14 within the external housing 10 is guided by the first and second rib pairs 21,22 bearing against and moving along the sealing surfaces 18,19.

In this embodiment as the removable part 50 of the cap 40 and inserter 14 continue to be displaced, the second pair of ribs pass the first step 17 and become axially misaligned with the second sealing surface 19 as shown in Figure 5(b). A flow path is thereby opened between the storage chamber 70 and a cavity defined between the catheter 120 and sheath 80, in this regard the interior of the sheath can be considered a wetting chamber 71. The wetting agent flows from the storage chamber 70 to the catheter 120 around the second rib pair 22. The apertures 32 in the gripper arms 31 ensure that wetting agent contacts the insertion end of the catheter 120. In this embodiment any excess wetting agent which does not contact the catheter flows through the sheath 80 and is discharged into the pouch.

In this embodiment as the storage chamber 70 is being opened the engagement tabs 58 and thus cap tabs 23 are drawn through the aperture 42. Although the cap tabs 23 are wider than the aperture 42 the chamfered step 60 resiliently deforms the aperture 42 increasing the diameter, this allows the cap tabs 23 to pass through the aperture 42. In alternate embodiments the cap tabs may instead, or in addition, be provided with chamfers, or be tapered such that a leading edge would have a reduced radial extent such that the cap tabs could deform the aperture.

The inserter 14 continues to move axially until the first pair of ribs 21 abut the end wall 64 at which point the gripper arms 31 have passed through the aperture 42 and are outside the external housing 10. Throughout the entire movement of the inserter 14 the first pair of ribs 21 remain in contact with the first sealing surface 18 ensuring no wetting agent leaks from the cap end 11 of the external housing 10.

As the gripper arms 31 are no longer radially nested within the aperture 42 or external housing 14 one or both can be deformed (either plastically or resiliently, depending upon the material) such that the cap tabs 23 are no longer within the apertures 59 and the cap can be removed as seen in Figure 5(c).

With the removable part 50 of the cap 40 removed, the inserter tip 15 is exposed, gripping the textured portion 13 the user can introduce the inserter tip 15 into the urethra, the extent to which this can be done being limited by the cap tabs 23. Though the inserter 14 is movable it does not retract completely back into the external housing when introduced into the urethra because having been resiliently deformed during deployment, the aperture 42 has returned to a diameter less that the separation between the radially outer faces 23a of the cap tabs 23 and as such the cap tabs 23 cannot return through the aperture 42. There may be a small amount of movement of the inserter 14 into the external housing from where the cap tabs 23 have been brought beyond the aperture 42 to allow the cap engagement tabs 58 to release.

Once the inserter tip 15 is in place the user can grip the catheter 120 through the sheath 80 and introduce it into the urethra and drain the bladder in the usual manner.

The one or more embodiments are described above by way of example only. Many variations are possible without departing from the scope of protection afforded by the appended claims.

For example, whilst the embodiments are all female intermittent urinary catheters, with an exemplary length of between 90mm to 200mm. e.g. between 130mm and 155mm, such as about 135mm and the catheter assemblies have a length corresponding to the length of the catheter, such as a closed length of the casing of between 2mm and 10mm longer than the length of catheter (e.g. 10-25cm; between 140mm and 165mm, such as 142mm), it is considered that teachings could be applied to male urinary intermittent catheters (which are typically longer) or even other types of catheter. Similarly, although the embodiments have functionalised hydrophilic surfaces which become slippery when wetted with a wetting agent such as water, the wetting agent could be a lubricant instead.

## Claims

1. A catheter assembly (1) comprising an external housing (10) and a cap (40);
wherein the external housing (10) and cap (40) form a cavity for housing at least part of a catheter (120);
the external housing (10) comprising a wetting agent storage chamber (70) defined by a chamber wall (9) and an inserter tip (15),
wherein the inserter tip (15) comprises an insertion end configured to be inserted into a urethra and the cap (40) comprises an interior cavity shaped to conform to a portion of the inserter tip (15)
wherein the inserter tip (15) is movable with respect to the chamber wall (9) between a first position and a second position;
wherein in the first position the inserter tip (15) is retracted and is aligned with the chamber wall (9) to seal the storage chamber (70);
and in the second position the inserter tip (15) is deployed and is misaligned with the chamber wall (9) to open the storage chamber (70); wherein removal of the cap (40) moves the inserter tip (15) from the first position to the second position.

2. A catheter assembly (1) according to claim 1 wherein one of the inserter tip (15) or chamber wall (9) comprises one or more ribs (21,22) extending circumferentially and extending to the other of the chamber wall (9) or inserter tip (15).

3. A catheter assembly (1) according to claim 2 wherein in the first position the one or more ribs (21,22) define an axial extent of the wetting agent storage chamber (70).

4. A catheter assembly (1) according to claim 3 or 4 wherein the inserter tip (15) comprises two ribs (21,22), the two ribs defining the axial extent of the storage chamber (70).

5. A catheter assembly (1) according to any of claims 2 to 4 wherein in the first position the one or more or each rib (21,22) aligns with a corresponding sealing surface (18,19) on the chamber wall (9) to seal the storage chamber (70).

6. A catheter assembly (1) according to any of claims 2 to 5 wherein in the second position at least one rib (22) of the inserter tip (15) is axially misaligned with a sealing surface (19) on the chamber wall (9).

7. A catheter assembly (1) according to claim 6 wherein the assembly comprises two ribs (21,22), an upper rib (21) remaining aligned with a sealing surface (18) on the chamber wall (9) in the second position.

8. A catheter assembly (1) according to any of claims 2 to 7, wherein at least one of the inserter tip (15) and the chamber wall (9) comprises a divergent portion (17) over which one or more or the or each rib (22) passes when transitioning between the first position and the second position such that the distance between the chamber wall (9) and inserter (15) at the axial location of the rib (22) is increased when in the second position.

9. A catheter assembly (1) according to claim 8 wherein the divergent portion (17) comprises a step or chamfer adjacent to the sealing surface (19).

10. A catheter assembly (1) according to any preceding claim further comprising a wetting agent, wherein when in the first position the wetting agent is sealed within the wetting agent storage chamber (70).

11. A catheter assembly (1) according to any preceding claim wherein in the second position there is provided a flow path from the wetting agent storage chamber (70) to the catheter (120) to wet the catheter (120).

12. A catheter assembly (1) according to any preceding claim, wherein the cap (40) is configured to disengage from the inserter tip (15) in the second position.

13. A catheter assembly (1) according to claim 12 wherein the cap (40) is deformable.

14. A catheter assembly (1) according to claim 13 wherein the cap (40) is deformed to disengage from the inserter tip (15).

15. A catheter assembly (1) according to claim 13 or 14 wherein in the first position the cap (40) is restricted from deforming by the external housing (10).

## Patentansprüche

1. Katheteranordnung (1) aufweisend ein Außengehäuse (10) und eine Kappe (40); wobei das Außengehäuse (10) und die Kappe (40) einen Hohlraum zur Aufnahme zumindest eines Teils eines Katheters (120) bilden; wobei das Außengehäuse (10) eine Benetzungsmittel-Speicherkammer (70) aufweist, die durch eine Kammerwand (9) und eine Einführspitze (15) definiert ist, wobei die Einführspitze (15) ein Einführungsende aufweist, das dazu ausgebildet ist, in eine Harnröhre eingeführt zu werden, und die Kappe (40) einen Innenhohlraum aufweist, der in seiner Form an einen Abschnitt der Einführspitze (15) anpasst ist, wobei die Einführspitze (15) in Bezug auf die Kammerwand (9) zwischen einer ersten Stellung und einer zweiten Stellung bewegbar ist; wobei in der ersten Stellung die Einführspitze (15) zurückgezogen und mit der Kammerwand (9) ausgerichtet ist, um die Speicherkammer (70) abzudichten; und in der zweiten Stellung die Einführspitze (15) ausgefahren und bezüglich der Kammerwand (9) versetzt ist, um die Speicherkammer (70) zu öffnen; wobei ein Entfernen der Kappe (40) die Einführspitze (15) von der ersten Stellung in die zweite Stellung bewegt.

2. Katheteranordnung (1) gemäß Anspruch 1, wobei eines aus der Einführspitze (15) oder der Kammerwand (9) eine oder mehrere Rippen (21, 22) aufweist, die sich in Umfangsrichtung und bis hin zum anderen aus der Kammerwand (9) oder der Einführspitze (15) erstrecken.

3. Katheteranordnung (1) gemäß Anspruch 2, wobei in der ersten Stellung die eine oder mehreren Rippen (21, 22) eine axiale Erstreckung der Benetzungsmittel-Speicherkammer (70) definieren.

4. Katheteranordnung (1) gemäß Anspruch 3 oder 4, wobei die Einführspitze (15) zwei Rippen (21, 22) aufweist, wobei die beiden Rippen die axiale Erstreckung der Speicherkammer (70) definieren.

5. Katheteranordnung (1) gemäß einem der Ansprüche 2 bis 4, wobei in der ersten Stellung die eine oder die mehreren oder jede Rippe (21, 22) mit einer korrespondierenden Dichtfläche (18, 19) an der Kammerwand (9) ausgerichtet ist, um die Speicherkammer (70) abzudichten.

6. Katheteranordnung (1) gemäß einem der Ansprüche 2 bis 5, wobei in der zweiten Stellung wenigstens eine Rippe (22) der Einführspitze (15) bezüglich einer Dichtfläche (19) an der Kammerwand (9) axial versetzt ist.

7. Katheteranordnung (1) gemäß Anspruch 6, wobei die Anordnung zwei Rippen (21, 22) aufweist, wobei eine obere Rippe (21) in der zweiten Stellung mit einer Dichtfläche (18) an der Kammerwand (9) ausgerichtet bleibt.

8. Katheteranordnung (1) gemäß einem der Ansprüche 2 bis 7, wobei wenigstens eine aus der Einführspitze (15) und der Kammerwand (9) einen divergierenden Abschnitt (17) aufweist, über sich den eine oder mehrere oder jede Rippe (22) beim Übergang zwischen der ersten Stellung und der zweiten Stellung hinwegbewegt, so dass sich in der zweiten Stellung der Abstand zwischen der Kammerwand (9) und der Einführspitze (15) an der axialen Position der Rippe (22) vergrößert.

9. Katheteranordnung (1) gemäß Anspruch 8, wobei der divergierende Abschnitt (17) eine Stufe oder Fase benachbart zur Dichtfläche (19) aufweist.

10. Katheteranordnung (1) gemäß einem der vorhergehenden Ansprüche, weiterhin aufweisend ein Benetzungsmittel, wobei das Benetzungsmittel in der ersten Stellung in der Benetzungsmittel-Speicherkammer (70) eingeschlossen ist.

11. Katheteranordnung (1) gemäß einem der vorhergehenden Ansprüche, wobei in der zweiten Stellung ein Strömungsweg von der Benetzungsmittel-Speicherkammer (70) zum Katheter (120) bereitgestellt ist, um den Katheter (120) zu benetzen.

12. Katheteranordnung (1) gemäß einem der vorhergehenden Ansprüche, wobei die Kappe (40) dazu ausgebildet ist, sich in der zweiten Stellung von der Einführspitze (15) zu lösen.

13. Katheteranordnung (1) gemäß Anspruch 12, wobei die Kappe (40) verformbar ist.

14. Katheteranordnung (1) gemäß Anspruch 13, wobei die Kappe (40) zum Lösen von der Einführspitze (15) verformt ist.

15. Katheteranordnung (1) gemäß Anspruch 13 oder 14, wobei in der ersten Stellung das Außengehäuse (10) ein Verformen der Kappe (40) verhindert.

## Revendications

1. Un ensemble (1) à cathéter comprenant un boîtier (10) extérieur et un capuchon (40) ; dans lequel le boîtier (10) extérieur et le capuchon (40) forment une cavité pour loger au moins une partie d'un cathéter (120) ; le boîtier (10) extérieur comprenant une chambre (70) de stockage d'un agent mouillant définie par une paroi (9) de chambre et un bout (15) d'inséreur,
dans lequel le bout (15) d'inséreur comprend une extrémité d'insertion configurée pour être insérée dans une urètre et le capuchon (40) comprend une cavité intérieure formée pour se conformer à une partie du bout (15) d'inséreur,
dans lequel le bout (15) d'inséreur est mobile par rapport à la paroi (9) de la chambre entre une première position et une deuxième position ;
dans lequel dans la première position, le bout (15) d'inséreur est escamoté et est aligné sur la paroi (9) de la chambre pour sceller la chambre (70) de stockage ; et dans la deuxième position, le bout (15) d'inséreur est déployé et est désaligné de la paroi (9) de la chambre pour ouvrir la chambre (70) de stockage ;
dans lequel le retrait du capuchon (40) déplace le bout (15) d'inséreur de la première position à la deuxième position.

2. Un ensemble (1) à cathéter suivant la revendication 1, dans lequel l'un du bout (15) d'inséreur ou de la paroi (9) de la chambre comprend une ou plusieurs nervures (21, 22) s'étendant circonférentiellement et s'étendant vers l'autre de la paroi (9) de la chambre ou du bout (15) d'inséreur.

3. Un ensemble (1) à cathéter suivant la revendication 2, dans lequel, dans la première position, la une ou les plusieurs nervures (21, 22) définissent une étendue axiale de la chambre (70) de stockage d'agent mouillant.

4. Un ensemble (1) à cathéter suivant la revendication 3 ou 4, dans lequel le bout (15) d'inséreur comprend deux nervures (21, 22), les deux nervures définissant l'étendue axiale de la chambre (70) de stockage.

5. Un ensemble (1) à cathéter suivant l'une quelconque des revendications 2 à 4, dans lequel, dans la première position, la une ou les plusieurs ou chaque nervure (21, 22) est alignée sur une surface (18, 19) correspondante de scellement sur la paroi (9) de la chambre pour sceller la chambre (70) de stockage.

6. Un ensemble (1) à cathéter suivant l'une quelconque des revendications 2 à 5, dans lequel, dans la deuxième position, au moins une nervure (22) du bout (15) d'inséreur est désalignée axialement d'une surface (19) de scellement sur la paroi (9) de la chambre.

7. Un ensemble (1) à cathéter suivant la revendication 6, dans lequel l'ensemble comprend deux nervures (21, 22), une nervure (21) supérieure restant alignée sur une surface (18) de scellement sur la paroi (9) de la chambre dans la deuxième position.

8. Un ensemble (1) à cathéter suivant l'une quelconque des revendications 2 à 7, dans lequel au moins l'un du bout (15) d'inséreur et de la paroi (9) de la chambre comprend une partie (17) divergente, sur laquelle une ou plusieurs ou la ou chaque nervure (22) passe, lors du passage entre la première position et la deuxième position, de manière à ce que la distance entre la paroi (9) de la chambre et l'inséreur (15) à l'emplacement axiale de la nervure (22) soit augmentée lorsque l'on est dans la deuxième position.

9. Un ensemble (1) à cathéter suivant la revendication 8, dans lequel la partie (17) divergente comprend un degré ou un chanfrein contigu à la surface (19) de scellement.

10. Un ensemble (1) à cathéter suivant l'une quelconque des revendications précédentes, comprenant en outre un agent mouillant, dans lequel, lorsque l'on est dans la première position, l'agent mouillant est scellé dans la chambre (70) de stockage d'agent mouillant.

11. Un ensemble (1) à cathéter suivant l'une quelconque des revendications précédentes, dans lequel, dans la deuxième position, il est prévu un chemin d'écoulement de la chambre (70) de stockage d'agent mouillant au cathéter (120) pour mouiller le cathéter (120).

12. Un ensemble (1) à cathéter suivant l'une quelconque des revendications précédentes, dans lequel le capuchon (40) est configuré pour se désengager du bout (15) d'inséreur dans la deuxième position.

13. Un ensemble (1) à cathéter suivant la revendication 12, dans lequel le capuchon (40) est déformable.

14. Un ensemble (1) à cathéter suivant la revendication 13, dans lequel le capuchon (40) est déformé pour se désengager du bout (15) d'inséreur.

15. Un ensemble (1) à cathéter suivant la revendication 13 ou 14, dans lequel, dans la première position, le capuchon (40) est empêché de se déformer par le boîtier (10) extérieur.
